(19) Europäisches Patentamt

European Patent Office

Office européen des brevets

(11) **EP 0 802 811 B1**

(12) **EUROPÄISCHE PATENTSCHRIFT**

(45) Veröffentlichungstag und Bekanntmachung des Hinweises auf die Patenterteilung:
**10.10.2001 Patentblatt 2001/41**

(21) Anmeldenummer: **95935794.8**

(22) Anmeldetag: **09.11.1995**

(51) Int Cl.$^7$: **A61M 5/315**, A61M 5/24

(86) Internationale Anmeldenummer:
**PCT/CH95/00262**

(87) Internationale Veröffentlichungsnummer:
**WO 97/17096 (15.05.1997 Gazette 1997/21)**

(54) **Injektionsvorrichtung**

Injection device

Dispositif d'injection

(84) Benannte Vertragsstaaten:
**AT BE CH DE DK ES FR GB GR IE IT LI LU MC NL PT SE**

(43) Veröffentlichungstag der Anmeldung:
**29.10.1997 Patentblatt 1997/44**

(73) Patentinhaber: **Disetronic Licensing AG**
**3400 Burgdorf (CH)**

(72) Erfinder: **MICHEL, Peter**
**CH-3400 Burgdorf (CH)**

(56) Entgegenhaltungen:
**EP-A- 0 554 995**  **WO-A-87/02895**
**WO-A-92/18179**  **DE-A- 3 900 926**

**Beschreibung**

Die Erfindung bezieht sich auf eine Vorrichtung zum wiederholten Injizieren von Dosen eines flüssigen Medikamentes gemäss dem Oberbegriff des Patentanspruches 1.

Solche Injektionsvorrichtungen sind seit längerer Zeit bekannt, so beispielsweise eine in der EP-0 245 312 B1 beschriebene Vorrichtung, die als nächstliegende Stand der Technik anzusehen ist. Sie dient zum Injizieren jeweils wählbarer Flüssigkeitsmengen aus einem mit einem Kolben ausgerüsteten Flüssigkeitsbehälter, insbesondere einer Karpule (= Spritzampulle).

Diese Karpule ist in einem Karpulenhalter an ein manuell antreibbares Getriebe an- und abbaubar. Das Getriebe ist in eine Getriebehülse eingebaut. Es enthält neben anderen Teilen:

- ein aus einem Führungsknopf und eine fest mit ihm verbundene Mitnehmerhülse bestehendes Antriebsglied, das drehbar und in der Vorschubrichtung des Kolbens hin- und herverschieblich in der Getriebehülse angebracht ist,
- ein in der Vorschubrichtung des Kolbens verschiebbares, aus einer Gewindestange und einem Flansch bestehendes Abtriebsglied, das von dem Antriebsglied bei Längsverschiebungen streckenweise mitnehmbar ist,
- ein Getriebeelement, das an der Gewindestange schraubbar gelagert ist, und
- eine Druckfeder, die zwischen dem Getriebeelement und der Mitnehmerhülse wirkt.

Diese Injektionseinrichtung kann im Betrieb folgende Stellungen einnehmen:

- eine Ruhestellung, in der das Antriebsglied weitmöglichst vom Kolben entfernt ist und das Getriebeelement nahe am Kolben liegt,
- eine Zwischenstellung, in der das Antriebsglied in seiner Stellung bleibt, bei der jedoch das Getriebeelement in einer von der zu injizierenden Flüssigkeitsmenge abhängigen Weise weiter vom Kolben entfernt liegt als bei der Ruhestellung und
- eine Endstellung, in der das Antriebsglied näher am Kolben liegt und das Abtriebsglied weiter als in der Ruhe- und der Zwischenstellung aus dem Getriebe herausgefahren und in die Karpule eingedrungen ist, was zur Injektion der vorgesehenen Flüssigkeitsmenge führte.
  Die Endstellung wird selbsttätig wieder in die Ruhestellung überführt.

In allen drei Stellungen ist das Getriebe nicht verriegelt, so dass es bei der Lagerung oder beim Transport der Injektionsvorrichtung Einwirkungen unterliegen kann, die zur Änderung der Flüssigkeitsmenge in der Karpule oder gar zum Eintreten von Luft in die Karpule führen kann. Darüber hinaus besitzt es viele Bauteile, die teilweise nicht ganz einfach herstellbar sind.

Der Erfindung liegt die Aufgabe zugrunde, eine Injektionsvorrichtung zu schaffen, die in mindestens einer Stellung, die dann als Endstellung gilt, selbsttätig verriegelbar ist.

Die erfindungsgemässe Lösung dieser Aufgabe ist Gegenstand des Patentanspruchs 1. Bevorzugte Weiterbildungen der Erfindung sind in den folgenden Ansprüchen beschrieben. Dabei kann die in diesen Ansprüchen beschriebene Injektionsvorrichtung mit weniger und zum Teil einfacher herzustellenden Teilen aufgebaut werden als vorbekannte Injektionsvorrichtungen.

Im Folgenden wird auf Grund der Zeichnung ein Ausführungsbeispiel der Erfindung näher erläutert. Es zeigen:

- Fig. 1 einen Achsenlängsschnitt durch die erfindungsgemässe Injektionsvörrichtung in einer Endstellung;
- Fig. 2 dasselbe in einer Ruhestellung; und
- Figur 3 dasselbe in einer Zwischenstellung.

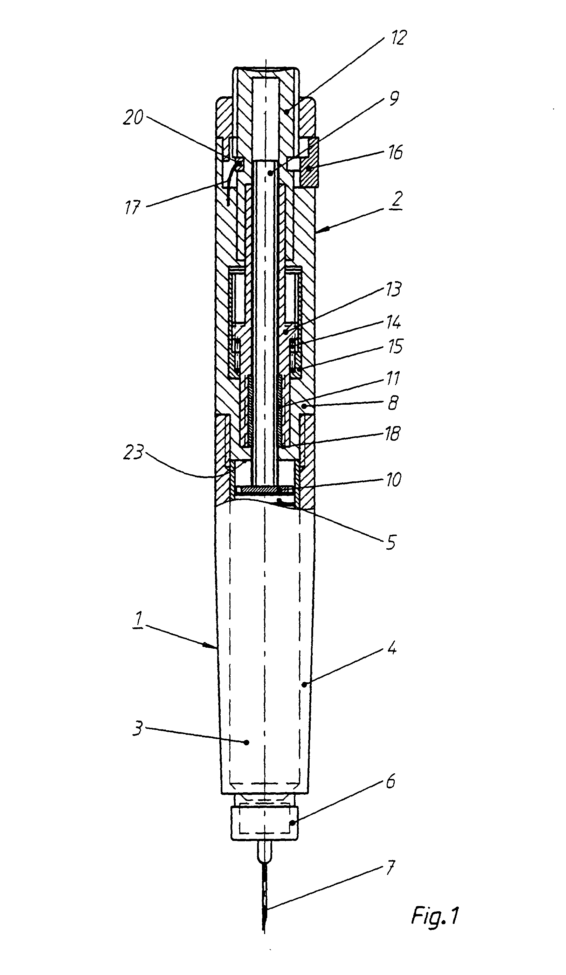

Die in den Figuren 1 bis 3 dargestellte Injektionsvorrichtung besitzt einen Karpulenteil 1 und ein Getriebe 2, die in einfacher Weise zusammenbaubar und trennbar sind, beispielsweise mit einem Gewinde oder einem Bayonettverschluss.

Der Karpulenteil 2 enthält einen Flüssigkeitsbehälter 3, im folgenden "Karpule" 3 genannt, der mit der zu injizierenden Flüssigkeit gefüllt ist, einen Karpulenhalter 4, eine Kolben 5, der stets auf der Flüssigkeit aufliegt und beim Injizieren die zu injizierende Flüssigkeit verdrängt, einen Nadelhalter 6 und eine Nadel 7, aus der die zu injizierende Flüssigkeit austritt.

Das Getriebe 2 ist in eine Getriebehülse 8 eingebaut. Es enthält:

- ein aus einem Führungsknopf 12 und einer fest mit ihm verbundene Mitnehmerhülse 13 bestehendes Antriebsglied 12, 13, das drehbar und in der Vorschubrichtung des Kolbens 5 verschieblich in der Getriebehülse 8 angebracht ist,
- ein in der Vorschubrichtung des Kolbens 5 verschiebbares, aus einer Gewindestange 9 und einem Flansch 10 bestehendes Abtriebsglied 9, 10, das unter der Wirkung des Längsbewegungen ausführenden Antriebsgliedes 12, 13 bei Längsverschiebungen streckenweise mitnehmbar ist,
- ein Getriebeelement 11, das die Mitnahme des Abtriebsgliedes 9, 10 durch das Antriebsglied 12, 13 vermittelt und das an der Gewindestange 9 schraubbar gelagert ist, und
- eine Druckfeder 14, die, durch eine Federhülse 15 gehalten, zwischen dem Getriebehülse 8 und der Mitnehmerhülse 13 wirkt.

Die erfindungsgemässe Injektionsvorrichtung besitzt folgende konstruktive Merkmale:

- In das Antriebsglied 12, 13 greift eine Verriegelungsvorrichtung 16, 17, 20, 21 ein.
- Die Gewindestange 9 des Abtriebgliedes 9, 10 ist drehfest, aber längsverschieblich in der Getriebehülse 8 eingebaut.
- Das Getriebeelement 11 ist drehfest, aber längsverschieblich mit dem Antriebsglied 12, 13 verbunden.
- Der Flansch 10 des Abtriebselementes 9, 10 liegt an dem stets auf der Flüssigkeit aufliegenden Kolben 5 an, so lange die Karpule 3 mit dem Getriebe 2 verbunden ist.

Sie weist die im folgenden beschriebenen Eigenschaften auf:

Nach dem Ende jeder Injektion befindet sich das Getriebe 2 jeweils in einer verriegelten Endstellung (Fig. 1), es kann in der Endstellung (Fig. 1) auch gelagert und transportiert werden. In dieser Endstellung (Fig. 1) ist das Antriebsglied 12, 13 gegenüber der Getriebehülse 8 so weit in der Vorschubrichtung in Richtung auf den Kolben 5 vorgeschoben, dass die Verriegelung 16, 17, 20, 21 zwischen der Getriebehülse 8 und dem Antriebsglied 12, 13 eingreift und dieses gegen Drehung und Längsverschiebung sichert. Ferner liegt das Getriebeelement 11 in Richtung des Kolbens 5 an einem ersten Anschlag 18 an der Getriebehülse 8 an, der diese in Richtung auf den Kolben zu begrenzt.

Bei eingegriffener Verriegelung 16, 17, 20, 21 ist das Antriebsglied 12, 13 weder drehbar noch verschieblich. Es können daher in der Endstellung (Fig. 1) - auch nicht willentlich - keinerlei Operationen mit der Injektionsvorrichtung durchgeführt werden und sie kann daher keine Flüssigkeit verlieren. Dies unterscheidet sie von vorbekannten Injektionsvorrichtungen.

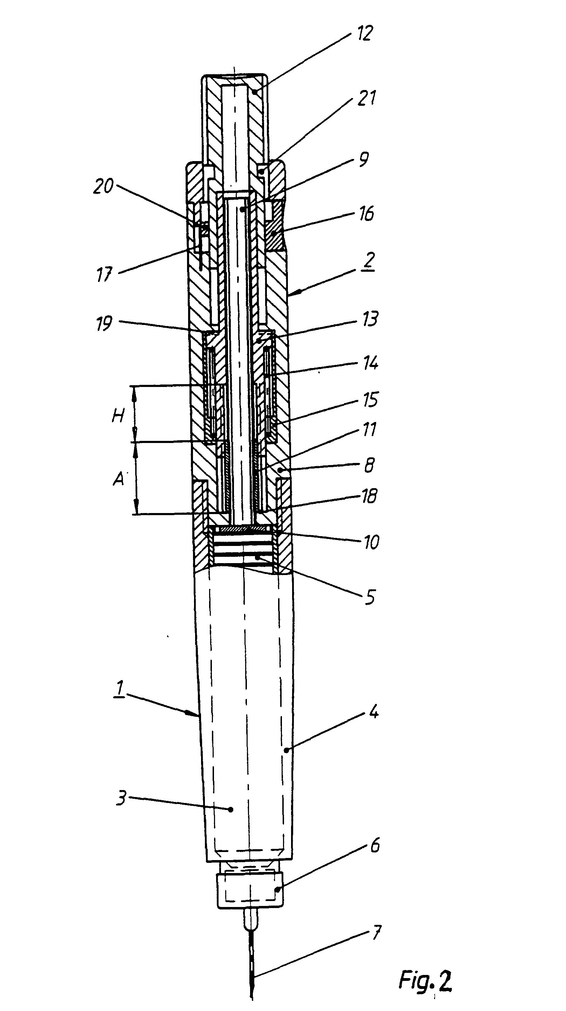

Die Verriegelung 16, 17, 20, 21 besteht beispielsweise aus einem das Antriebsglied 12, 13 umfassenden Verriegelungsring 16 und einem damit verbundenen Nocken 20. Dieser sitzt in der Getriebehülle 8 und greift, durch eine Feder 17 gedrückt, in der Endstellung (Fig. 1) in das Antriebsglied 12, 13 ein. Der Nocken kann aus der Nut 21 nur durch Druck gegen die Feder 17 auf den Verriegelungsring 16 gelöst werden. Ist die Verriegelung 16, 17, 20, 21 gelöst, so ist das Antriebsglied 12, 13 wieder dreh- und verschiebbar und wird durch die Druckfeder 14 selbsttätig in die später beschriebene Ruhestellung (Fig. 2) verschoben. Eine Verriegelung, die vergleichbare Eigenschaften aufweist, kann aber auch auf andere Weise aufgebaut sein.

Um eine neue Injektion auszuführen, wird das Getriebe 2 zunächst in eine Ruhestellung (Fig. 2) gebracht. In dieser ist das Antriebsglied 12, 13 entgegen der Vorschubrichtung des Kolbens 5 weitmöglichst von diesem weggeschoben, was durch die Druckfeder 14 geschieht, die es um die Weglänge H bis zu einem zweiten Anschlag 19 an der Getriebehülse 8 schiebt. Der zweite Anschlag 19 ist beispielsweise ein Ringwulst auf der Innenmantelfläche der Getriebehülse 8. Das Getriebeelement 11 steht immer noch in Richtung des Kolbens 5 am ersten Anschlag 18 der Getriebehülse 8 an. Der Übergang von der Endstellung (Fig. 1) in die Ruhestellung (Fig. 2) wird durch Lösen der Verriegelung (16,17,20,21) der Getriebehülse 8 und dem Antriebsglied 12, 13 ausgeführt, wobei das Antriebsglied 12, 13 drehbar und längsverschiebbar wird und die Druckfeder 14 das Antriebsglied 12, 13 entgegengesetzt zur Vorschubrichtung des Kolbens 5 in die Ruhestellung (Fig. 2) verschiebt.

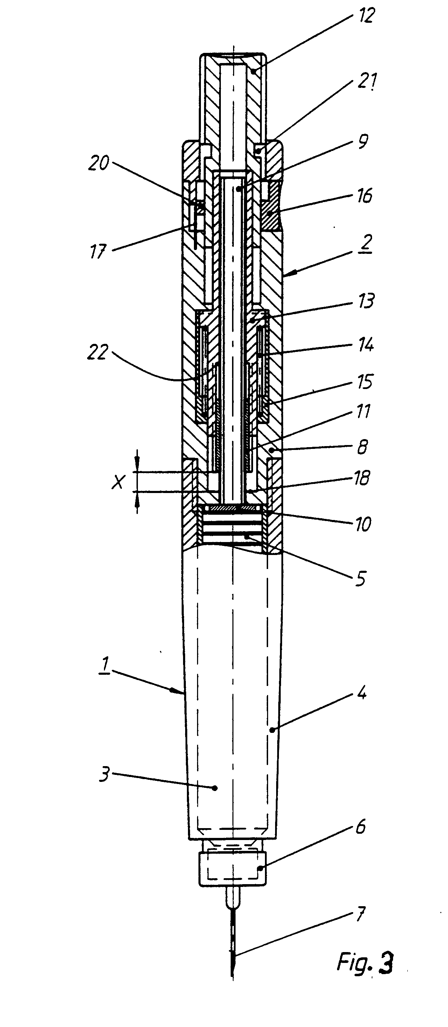

Von der Ruhestellung (Fig. 2) aus wird das Getriebe 2 in eine Zwischenstellung (Fig. 3) gebracht, durch welche die bei der nächsten Injektion zu injizierende Flüssigkeitsmenge vorgegeben ist. In ihr ist das untere Ende des Getriebeelements 11 entgegengesetzt zur Vorschubrichtung des Kolbens 5 um einen Abstand X gegenüber dem ersten Anschlag 18 an der Getriebehülse 8 verschoben, wobei der Abstand X bestimmt wird durch:

$$X = I/F$$

mit I = zu injizierende Flüssigkeitsmenge

     F = innere Querschnittsfläche der Karpule.

Der Übergang von der Ruhestellung (Fig. 2) in die Zwischenstellung (Fig. 3) wird durch Drehen des Antriebsgliedes 12, 13 in einem solchen Drehsinn bewirkt, dass das längsverschieblich und drehbar mit dem Antriebsglied 12, 13 verbundene Getriebeelement 11 um seine Achse, die Gewindestange 9, so mitgedreht wird, dass es längs der Gewindestange 9 entgegen der Vorschubrichtung des Kolbens 5 um den Abstand X verschoben wird.

Das Getriebeelement 11 ist maximal bis zu einem dritten Anschlag 22 an der Mitnehmerhülse 13 verschiebbar. Dieser liegt gegen die Vorschubrichtung des Kolbens an der Innenmantelfläche des Antriebsgliedes in einem Abstand A + H von dem ersten Anschlag 18 entfernt, wobei

    A = Länge des Getriebeelements (11),

    H = Weglänge des Antriebsgliedes zwischen der Endstellung (Fig. 1) und der Ruhestellung (Fig. 2) ist.

Für den maximalen Verschiebeweg H gilt H ≥ X.

Aus der Zwischenstellung (Fig. 3) wird das Getriebe in die Endstellung (Fig. 1) gebracht. Dabei läuft der Injektionsvorgang ab. Dazu wird das Antriebsglied 12, 13 um die Weglänge H in der Vorschubrichtung des Kolbens 5 vorgeschoben. Das Antriebsglied 12, 13 nimmt beim Vorschieben das in Entfernung X vom ersten Anschlag 18 an der Getriebehülse 8 stehende Getriebeelement 11 nach vorn mit. Von dann an nimmt das Getriebeelement 11 das nur längsverschiebliche Abtriebglied 9, 10 über die Weglänge X mit. Dabei schiebt dessen Flansch 10 den Kolben 5 in der Karpule 3 vor, so dass insgesamt die Flüssigkeitsmenge I = F x X verdrängt wird und durch die Nadel 7 austritt (Injektion). Haben das Antriebsglied 12, 13 die Weglänge H und das Getriebeelement 11 gleichzeitig die Weglängen X durchlaufen, so greift die Verriegelung 16, 17, 20, 21 in das Antriebsglied 12, 13 ein. Somit nimmt das Getriebe 2 die Endstellung ein, die Antriebseinrichtung 12,13 ist keiner Drehungen oder Längsverschiebungen mehr fähig und somit die Injektionsvorrichtung gegen einen Flüssigkeitsverlust geschützt.

Nach dem Verbrauch der gesamten Flüssigkeit der Karpule 3 wird in der Ruhestellung (Fig. 2) der Karpulenteil 1 von dem Getriebe 2 abgetrennt. Dann muss durch Drehen des Antriebsgliedes 12,13 in umgekehrtem Drehsinn wie beim Übergang von der Ruhestellung (Fig. 2) in die Zwischenstellung (Fig. 3) das Abtriebsglied 9, 10 so weit zurückgesetzt werden, bis der Flansch 10 an einem durch das dem Kolben 5 zugewandte Ende der Getriebehülse 8 gebildeten Auschlag 23 anschlägt.

Vorteilhafterweise sind neu einzusetzende Karpulen 3 so gefüllt, dass der Kolben 5 beim Einbau in die Injektionsvorrichtung durch den Flansch 10 ein wenig vorgeschoben wird und etwas Flüssigkeit aus der Nadel 7 austritt. Dadurch wird gewährleistet, dass auch bei der ersten Injektion mit der neuen Karpule 3 keine Luft mitinjiziert wird.

Erfindungsgemässe Injektionsvorrichtungen können in bekannter Weise elektrische, optische oder akustische Einrichtungen besitzen, mit der die eingestellte Weglänge X anzeigbar ist. dies kann beispielsweise eine Ratsche sein, die zwischen dem Antriebsglied 9, 10 und der Getriebehülse 8 wirkt.

Die erfindungsgemässe Injektionsvorrichtung besitzt eine Verriegelung 16, 17, 20, 21, die verhindert, dass in der Endstellung (Fig. 1) auch nicht willentliche Operationen mit ihr vorgenommen werden können. Dies bedeutet, dass sie mit angebauter Karpule 3 zuverlässiger gelagert und transportiert werden kann als die Vorrichtungen nach dem Stand der Technik. Darüber hinaus sind zu ihrem Aufbau weniger und teilweise in einfacher Weise herstellbare Bauteile erforderlich, als bei diesen, so dass sie weniger Herstellkosten erfordert.

## Patentansprüche

1. Injektionsvorrichtung zum Injizieren jeweils wählbarer Flüssigkeitsmengen aus einem mit einem Kolben (5) ausgerüsteten Flüssigkeitsbehälter (3), insbesondere einer Karpule (3), mit einem manuell antreibbaren Getriebe (2), an das der Flüssigkeitsbehälter (3) anbaubar und von dem der Flüssigkeitsbehälter abbaubar ist, das in eine Getriebehülse (8) eingebaut ist, und das enthält:

- ein aus einem Führungsknopf (12) und einer fest mit ihm verbundenen Mitnehmerhülse (13) bestehendes Antriebsglied (12, 13), das drehbar und in der Vorschubrichtung des Kolbens (5) verschieblich in der Getriebehülse (8) angebracht ist,
- ein in der Vorschubrichtung des Kolbens (5) verschiebbares, aus einer Gewindestange (9) und einem Flansch (10) bestehendes Abtriebsglied (9, 10), das unter der Wirkung des Längsverschiebungen ausführenden Antriebsglieds (12, 13) streckenweise mitnehmbar ist,
- ein Getriebeelement (11), das an der Gewindestange (9) schraubbar gelagert ist und die Mitnahme des Abtriebsgliedes (9, 10) durch das Antriebsglied (12, 13) vermittelt und
- eine Druckfeder (14), die zwischen der Getriebehülse (8) und der Mitnehmerhülse (13) wirkt,

**dadurch gekennzeichnet, dass**

- das Antriebsglied durch eine Verriegelungsvorrichtung (16, 17, 20, 21) feststellbar ist,
- die Gewindestange (9) des Abtriebgliedes (9, 10) drehfest, aber längsverschieblich in der Getriebehülse (8) gelagert ist und
- das Getriebeelement (11) drehfest und längsverschieblich mit dem Antriebsglied (12, 13) verbunden ist.

2. Injektionsvorrichtung nach Anspruch 1, **dadurch gekennzeichnet, dass** der Flansch (10) des Abtriebsgliedes (9, 10) an dem stets auf der Flüssigkeit aufliegenden Kolben (5) anliegt, so lange die Karpule (3) mit dem Getriebe (2) verbunden ist.

3. Injektionsvorrichtung nach Anspruch 1 oder 2, **dadurch gekennzeichnet, dass** das Getriebe (2) drei Stellungen einnehmen kann, eine Endstellung (Fig. 1), eine Ruhestellung (Fig. 2) und eine Zwischenstellung (Fig. 3).

4. Injektionsvorrichtung nach Anspruch 3, **dadurch gekennzeichnet, dass** in der Endstellung (Fig 1):

- das Antriebsglied (12, 13) gegenüber der Getriebehülse (8) so weit in der Vorschubrichtung gegen den Kolben (5) vorgeschoben ist, dass eine Verriegelung (16, 17, 20, 21) zwischen der Getriebehülse (8) und dem Antriebsglied (12, 13) eingreift und dieses gegen Drehung und Längsverschiebung sichert, und
- das Getriebeelement (11) in Richtung des Kolbens (5) an einem ersten Anschlag (18) an der Getriebehülse (8), der diese in Richtung auf den Kolben zu begrenzt, anliegt.

5. Injektionsvorrichtung nach Anspruch 3 oder 4, **da-**

**durch gekennzeichnet, dass** in der Ruhestellung (Fig. 2) das Antriebsglied (12, 13) entgegen der Vorschubrichtung des Kolbens (5) durch die Druckfeder (14) so verschoben ist, dass es an einem zweiten Anschlag (19) an der Getriebehülse (8) anschlägt, und

das Getriebeelement (11) in Richtung des Kolbens (5) am ersten Anschlag (18) der Getriebehülse (8) stehen bleibt.

6. Injektionsvorrichtung nach Anspruch 4 oder 5, **dadurch gekennzeichnet, dass** die Verriegelung (16, 17, 20, 21) aus einem Verriegelungsring (16) und einem Nocken (20) besteht, die in der Getriebehülse (8) sitzen und durch eine Feder (17) gegen das Antriebsglied (12, 13) drücken, wobei der Nocken (20) in der Endstellung (Fig. 1) in einer Nut (21) am Antriebsglied (12) eingreift und aus der Nut (21) nur durch Druck auf den Verriegelungsring (16) gelöst werden kann.

7. Injektionsvorrichtung nach einem der Ansprüche 3 - 7, **dadurch gekennzeichnet, dass** in einer einstellbaren Zwischenstellung (Fig. 3) das untere Ende des Getriebeelements (11) entgegen der Vorschubrichtung des Kolbens (5) gegenüber dem ersten Anschlag (18) an der Getriebehülse (8) um eine Weglänge X verschoben ist.

8. Injektionsvorrichtung nach Anspruch 7, **dadurch gekennzeichnet, dass** die Weglänge X bestimmt wird durch:

$$X = I/F$$

mit I = zu injizierende Flüssigkeitsmenge,
F = innere Querschnittsfläche der Karpule.

9. Injektionsvorrichtung nach Anspruch 8 oder 9, **dadurch gekennzeichnet, dass** das Getriebeelement (11) maximal bis zu einem dritten Anschlag (22) an der Mitnehmerhülse (13) verschiebbar ist, wobei für den maximalen Verschiebeweg H gilt:

$$H \geq X$$

dabei ist H die Weglänge der Verschiebung des Antriebsgliedes (12, 13) zwischen der Endstellung (Fig. 1) und der Ruhestellung (Fig.2).

10. Injektionsvorrichtung nach einem der Ansprüche 4 - 9, **dadurch gekennzeichnet, dass** in der Zwischenstellung (Fig. 3)

- das Antriebsglied (12,13) in der Vorschubrichtung des Kolbens (5) längsverschiebbar ist,

- das in einer Entfernung vom ersten Anschlag (18) an der Getriebehülse (8) stehende Getriebeelement (11) vom Antriebsglied (12,13) eine bestimmte Wegstrecke längsverschieblich mitnehmbar ist,

- eine Längsbewegung des Getriebeelements (11) unmittelbar auf das Abtriebsglied (9, 10) wirkt,

- die Längsbewegung des Getriebeelementes (11) am ersten Anschlag (18) an der Getriebehülse (8) begrenzt ist,

- auf dieser Wegstrecke der Flansch (10) des Abriebgliedes (9, 10) den Kolben (5) in die Karpule (3) drückt, so dass insgesamt die zu injizierende Flüssligkeitsmenge durch die Nadel austritt,

- bei Erreichen des ersten Anschlags (18) an der Getriebehülse (8) durch das Getriebeelement (11) die Verriegelung (16, 17, 20, 21) in das Antriebsglied (12, 13) eingreift.

11. Injektionsvorrichtung nach einem der Ansprüche 1 - 10, **dadurch gekennzeichnet, dass** das Antriebsglied (12, 13) in umgekehrtem Drehsinn drehbar ist und dadurch das Abtriebsglied (9, 10) so weit zurücksetzbar ist, bis der Flansch (10) an einem durch das dem Kolben (5) zugewandten Ende der Getriebehülse (8) gebildeten Auschlag (23) anschlägt.

12. Injektionsvorrichtung nach einem der Ansprüche 1 - 11, **dadurch gekennzeichnet, dass** beim Anbau einer vollen Karpule (3) an das Getriebe (2) der Flansch (10) den Kolben (5) ein wenig verschiebt, dadurch ein wenig Flüssigkeit aus der Nadel (7) austritt.

13. Injektionsvorrichtung nach einem der Ansprüche 1 - 12, **dadurch gekennzeichnet, dass** sie eine elektrische, optische oder akustische Einrichtung besitzt, mit der die eingestellte Weglänge X anzeigbar ist.

14. Injektionsvorrichtung nach Anspruch 13, **dadurch gekennzeichnet, dass** die Akustische Einrichtung eine Ratsche ist, die zwischen dem Antriebsglied (12, 13) und der Getriebehülse (8) wirkt.

**Claims**

1. An injection device for injecting respective selectable amounts of liquid from a liquid container (3), in particular an injection ampoule (3), which is equipped with a plunger (5), comprising a manually drivable transmission (2) to which the liquid container (3) can be fitted and from which the liquid container can be removed, which is installed in a transmission sleeve (8) and which includes:

- a drive member (12, 13) which comprises a guide knob (12) and an entrainment sleeve (13) which is fixedly connected thereto, the drive member being mounted in the transmission sleeve (8) rotatably and slidably in the advance direction of the plunger (5),
- an output member (9, 10) which is displaceable in the advance direction of the plunger (5) and which comprises a screwthreaded rod (9) and a flange (10) and which can be entrained in a section-wise movement under the action of the drive member (12, 13) implementing longitudinal sliding movements,
- a transmission element (11) which is mounted screwably on the screwthreaded rod (9) and which provides for entrainment of the output member (9, 10) by the drive member (12, 13), and
- a compression spring (14) which is operative between the transmission sleeve (8) and the entrainment sleeve (13),

    **characterised in that**

- the drive member can be fixed by a locking device (16, 17, 20, 21),
- the screwthreaded rod (9) of the output member (9, 10) is mounted non-rotatably but longitudinally slidably in the transmission sleeve (8), and
- the transmission element (11) is connected non-rotatably and longitudinally slidably to the drive member (12, 13).

2. An injection device according to claim 1 **characterised in that** the flange (10) of the output member (9) bears against the plunger (5) which always rests on the liquid, as long as the injection ampoule is connected to the transmission (2).

3. An injection device according to claim 1 or claim 2 **characterised in that** the transmission (2) can assume three positions, an end position (Figure 1), a rest position (Figure 2) and an intermediate position (Figure 3).

4. An injection device according to claim 3 **characterised in that** in the end position (Figure 1):

- the drive member (12, 13) is advanced towards the plunger (5) in the advance direction with respect to the transmission sleeve (8), to such an extent that a locking means (16, 17, 20, 21) engages between the transmission sleeve (8) and the drive member (12, 13) and prevents same from rotation and longitudinal sliding movement, and

- the transmission element (11) bears in the direction of the plunger (5) against a first abutment (18) on the transmission sleeve (8) which delimits same in a direction towards the plunger.

5. An injection device according to claim 3 or claim 4 **characterised in that** in the rest position (Figure 2) the drive member (12, 13) is displaced in opposite relationship to the advance direction of the plunger (5) by the compression spring (14) so that it bears against a second abutment (19) on the transmission sleeve (8), and the transmission element (11) remains against the first abutment (18) of the transmission sleeve (8) in the direction of the plunger (5).

6. An injection device according to claim 4 or claim 5 **characterised in that** the locking means (16, 17, 20, 21) comprises a locking ring (16) and a projection (20) which sit in the transmission sleeve (8) and press by means of a spring (17) against the drive member (12, 13), wherein in the end position (Figure 1) the projection (20) engages in a groove (21) on the drive member (12) and can be released from the groove (21) only by pressure on the locking ring (16).

7. An injection device according to one of claims 3 to 6 **characterised in that** in a settable intermediate position (Figure 3) the lower end of the transmission element (11) is displaced by a travel length X in opposite relationship to the advance direction of the plunger (5) with respect to the first abutment (18) on the transmission sleeve (8).

8. An injection device according to claim 7 **characterised in that** the travel length X is determined by:

$$X = I/F$$

with I = amount of liquid to be injected, and F = internal cross-sectional area of the injection ampoule.

9. An injection device according to claim 8 **characterised in that** the transmission element (11) is displaceable at a maximum to a third abutment (22) on the entrainment sleeve (13), wherein the following applies for the maximum displacement travel H:

$$H \geq X$$

wherein H is the travel length of the displacement of the drive member (12, 13) between the end position (Figure 1) and the rest position (Figure 2).

10. An injection device according to one of claims 4 to 9 **characterised in that** in the intermediate position (Figure 3)

- the drive member (12, 13) is longitudinally slidable in the advance direction of the plunger (5),
- the transmission element (11) which is at a distance from the first abutment (18) on the transmission sleeve (8) can be longitudinally slidably entrained a given travel distance by the drive member (12, 13),
- a longitudinal movement of the transmission element (11) acts directly on the output member (9, 10),
- the longitudinal movement of the transmission element (11) is delimited at the first abutment (18) on the transmission sleeve (8),
- over that travel distance the flange (10) of the output member (9, 10) presses the plunger (5) into the injection ampoule (3) so that overall the amount of liquid to be injected issues through the needle, and
- when the first abutment (18) on the transmission sleeve (8) is reached by the transmission element (11) the locking means (16, 17, 20, 21) engages into the drive member (12, 13).

11. An injection device according to one of claims 1 to 10 **characterised in that** the drive member (12, 13) is rotatable in the reverse direction of rotation and **in that** way the output member (9, 10) can be set back to such an extent until the flange (10) bears against an abutment (23) formed by the end, towards the plunger (5), of the transmission sleeve (8).

12. An injection device according to one of claims 1 to 11 **characterised in that** when a full injection ampoule (3) is fitted to the transmission (2) the flange (10) displaces the plunger (5) a little and thereby a little liquid issues from the needle (7).

13. An injection device according to one of claims 1 to 12 **characterised in that** it has an electrical, optical or acoustic device with which the set travel length X can be displayed.

14. An injection device according to claim 13 **characterised in that** the acoustic device is a ratchet which is operative between the drive member (12, 13) and the transmission sleeve (8).

**Revendications**

1. Dispositif d'injection, pour injecter des quantités de liquide à choisir au coup par coup, à partir d'un réservoir de liquide (3), en particulier d'une cartouche (3), équipé d'un piston (5), comprenant une transmission (2) sur laquelle le réservoir de liquide (3) est susceptible d'être monté et depuis laquelle le réservoir de liquide est susceptible d'être démonté, ladite transmission étant intégrée dans un manchon de transmission (8) et comprenant :

- un organe menant (12, 13) constitué par un bouton de guidage (12) et par un manchon d'entraînement (13) fermement relié à celui-ci, ledit organe menant étant monté dans le manchon de transmission (8) en rotation et en translation dans la direction de progression du piston (5),
- un organe mené (9, 10), en translation dans la direction de progression du piston (5) et constitué par une tige filetée (9) et par une bride (10), ledit organe mené étant susceptible d'être entraîné par tronçons sous l'action de l'organe menant (12, 13) qui exécute des translations longitudinales,
- un élément de transmission (11), monté par vissage sur la tige filetée (9) et transmettant l'entraînement de l'organe mené (9, 10) par l'organe menant (12, 13), et
- un ressort de compression (14), qui agit entre le manchon de transmission (8) et le manchon d'entraînement (13),

**caractérisé en ce que** :

- l'organe menant est susceptible d'être fixé par un dispositif de verrouillage (16, 17, 20, 21),
- la tige filetée (9) de l'organe mené (9, 10) est montée solidairement en rotation dans le manchon de transmission (8), mais avec faculté de translation longitudinale, et
- l'élément de transmission (11) est relié solidairement en rotation et avec faculté de translation longitudinale à l'organe menant (12, 13).

2. Dispositif d'injection selon la revendication 1, **caractérisé en ce que** la bride (10) de l'organe mené (9, 10) est appuyée sur le piston (5) en contact permanent avec le liquide aussi longtemps que la cartouche (3) est reliée à la transmission (2).

3. Dispositif d'injection selon la revendication 1 ou 2, **caractérisé en ce que** la transmission (2) peut occuper trois positions, à savoir une position finale (figure 1), une position de repos (figure 2) et une position intermédiaire (figure 3).

**4.** Dispositif d'injection selon la revendication 3, **caractérisé en ce que**, dans la position finale (figure 1) :

- l'organe menant (12, 13) est déplacé par rapport au manchon de transmission (8) dans la direction de progression et contre le piston (5), aussi loin qu'un verrouillage (16, 17, 20, 21) s'engage entre le manchon de transmission (8) et l'organe menant (12, 13) et bloque celui-ci à l'encontre d'une rotation et d'une translation longitudinale, et
- l'élément de transmission (11) s'appuie en direction du piston (5) contre une première butée (18) sur le manchon de transmission (8) qui limite celui-ci en direction du piston.

**5.** Dispositif d'injection selon la revendication 3 ou 4, **caractérisé en ce que** dans la position de repos (figure 2) l'organe menant (12, 13) est déplacé à l'encontre de la direction de progression du piston (5) par le ressort de compression (14) de telle manière qu'il vient buter contre une deuxième butée (19) sur le manchon de transmission (8), et l'élément de transmission (11) reste arrêté en direction du piston (5) au niveau de la première butée (18) du manchon de transmission (8).

**6.** Dispositif d'injection selon la revendication 4 ou 5, **caractérisé en ce que** le verrouillage (16, 17, 20, 21) est constitué d'une bague de verrouillage (16) et d'une came (20), lesquelles sont logées dans le manchon de transmission (8) et poussent au moyen d'un ressort (17) contre l'organe menant (12,13), et **en ce que**, dans la position finale (figure 1), la came (20) s'engage dans une gorge (21) sur l'organe menant (12) et ne peut être libérée hors de la gorge (21) que par pression sur la bague de verrouillage (16).

**7.** Dispositif d'injection selon l'une quelconque des revendications 3 à 6, **caractérisé en ce que** dans une position intermédiaire réglable (figure 3), l'extrémité inférieure de l'élément de transmission (11) est déplacée à l'encontre de la direction de progression du piston (5) d'une distance X par rapport à la première butée (18) sur le manchon de transmission (8).

**8.** Dispositif d'injection selon la revendication 7, **caractérisé en ce que** la course X est déterminée par la relation :

X = I/F, dans laquelle
I = quantité de liquide injecté, et
F = surface de section intérieure de la cartouche.

**9.** Dispositif d'injection selon la revendication 7 ou 8, **caractérisé en ce que** l'élément de transmission (11) est déplaçable au maximum jusqu'à une troisième butée (22) sur le manchon d'entraînement (13), et la course de déplacement maximum H satisfait la relation H ≥ X dans laquelle H est la longueur de la course de translation de l'organe menant (12, 13) entre la position finale (figure 1) et la position de repos (figure 2).

**10.** Dispositif d'injection selon l'une quelconque des revendications 4 à 9, **caractérisé en ce que**, dans la position intermédiaire (figure 3) :

- l'organe menant (12, 13) est capable de se déplacer en translation longitudinale dans la direction de progression du piston (5),
- l'élément de transmission (11) disposé à distance de la première butée (18) sur le manchon de transmission (8) peut être entraîné en translation longitudinale sur un certain trajet par l'organe menant (12, 13),
- un mouvement longitudinal de l'élément de transmission (11) agit directement sur l'organe mené (9,10),
- le mouvement longitudinal de l'élément de transmission (11) est limité au niveau de la première butée (18) sur le manchon de transmission (8),
- sur ce trajet, la bride (10) de l'organe mené (9, 10) pousse le piston (9) dans la cartouche (3), de sorte que la totalité de la quantité de liquide à injecter sort par l'aiguille, et
- lorsque l'élément de transmission (11) atteint la première butée (18) sur le manchon de transmission (8), le verrouillage (16, 17, 20, 21) s'engage dans l'organe menant (12, 13).

**11.** Dispositif d'injection selon l'une quelconque des revendications 1 à 10, **caractérisé en ce que** l'organe menant (12, 13) est susceptible de tourner dans le sens de rotation opposé, et que de ce fait l'organe mené (9, 10) est susceptible d'être reculé aussi loin que la bride (10) vient buter contre une butée (23) formée sur l'extrémité de la douille de transmission (8) tournée vers le piston (5).

**12.** Dispositif d'injection selon l'une quelconque des revendications 1 à 11, **caractérisé en ce que** lors du montage d'une cartouche pleine (3) sur la transmission (2), la bride (10) déplace légèrement le piston (5) et que par conséquent un peu de liquide sort de l'aiguille (7).

**13.** Dispositif d'injection selon l'une quelconque des revendications 1 à 12, **caractérisé en ce qu'**il possède des moyens électriques, optiques ou acoustiques, au moyen desquels peut être indiquée la course X établie.

14. Dispositif d'injection selon la revendication 13, **caractérisé en ce que** les moyens acoustiques sont formés par un cliquet agissant entre l'organe menant (12, 13) et la douille de transmission (8).

*Fig.1*

*Fig. 2*

Fig. 3